# EUROPEAN PATENT SPECIFICATION

(11) **EP 2 106 821 B1**
(45) Date of publication and mention of the grant of the patent: **17.04.2019**
(21) Application number: 08153767.2
(22) Date of filing: 31.03.2008
(51) Int. Cl.: A61M 25/00, A61M 39/08, A61M 27/00

(54) **Catheter assembly comprising a tubular member having pleated regions, and methods of activating and manufacturing the same**
Katheteranordnung mit einem rohrförmigen Mittel mit gefalteten Bereichen und Verfahren zur Aktivierung und Herstellung davon
Ensemble de cathéter comportant un élément tubulaire disposant de régions plissées, et son procédé d'activation et de fabrication

(43) Date of publication of application: 07.10.2009
(73) Proprietor: Dentsply IH AB, 431 21 Mölndal (SE)
(72) Inventor: Kedem, Sonja, 421 60, VÄSTRA FRÖLUNDA (SE); Nordholm, Agneta, 433 51 Öjersjö (SE)
(74) Representative: AWA Sweden AB

(56) References cited:
- EP-A- 0 423 855
- EP-A- 1 023 882
- WO-A-2006/074159
- DE-U1-202005 009 946
- US-A- 4 568 334

## Description

### Technical Field of the Invention

The present invention relates to a catheter assembly comprising a catheter having an insertion end and a discharge end, and a receptacle, which receptacle comprises a tubular member which in a storage state accommodates a catheter section including the insertion end of the catheter.

The present invention also relates to a method for activation of such catheter assembly as well as a method for manufacturing the same.

### Background Art

Catheters find their use in many different medical applications, such as urinary catheters for bladder drainage. These urinary catheters are inserted in a variety of populations, including the elderly, stroke victims, spinal cord-injured patients, post-operative patients and those with obstructive uropathy.

In designing a catheter, consideration needs to be taken to the intended use. Consequently, the material of which the catheter consists of commonly fulfils requirement such as softness, good kink resistance, good dimensional stability, and the possibility to be sterilized by radiation, steam, ethylene oxide or other means. For some catheters, there is further the need for the material to accept a surface treatment which will impart desired surface properties to the medical device, such as hydrophilicity.

Furthermore, in order to maintain the catheter in a clean and preferably sterile condition, each catheter is normally pre-packed in a receptacle by the manufacturer, thereby providing a catheter assembly.

As a catheter needs to be of sufficient length to accommodate the urethra, for instance having an insertable length of 200-350 mm for male users, the catheter assembly generally requires more space than is convenient. The assembly may thus be cumbersome and expensive to store, transport and handle, which is inconvenient not least for the individuals for whom catherisation is a daily-life procedure. To comply with the desire for less space consuming catheters, there has been a strive for catheters being space reducing. Such catheters allow for reduced space consumption during transport and also for improved life quality for the user of catheters, as the catheter assemblies may be stored more discreetly, for instance in the pocket of a users clothing.

The provision of a catheter assembly which during storage is arranged in a curved manner, however, may impose difficulties due to the catheter being of a material having certain rigidity / shape memory characteristics. Storing the catheter assembly for a long period of time in a non-straight disposition, may result in kinking and distortion of the catheter, and imply resistance of easily reverting the catheter to its straight disposition prior to an intended use. The user may subsequently experience discomfort during insertion of the catheter, especially if the first section of the insertable part remains in a non-straight disposition.

WO 2004/02243, for instance, discloses an elongated surgical device, exemplified as a catheter, arranged in a coiled disposition within a package, and with welds arranged to keep the elongate member in the coiled disposition. Furthermore US 5,344,011 relates to a packaging system for keeping for instance a catheter in a coiled disposition during storage, and in a similar manner, US 6,053,313 discloses another type of packaging for a catheter for maintaining the catheter in a coiled position.

EP 1 023 882 discloses a urinary catheter assembly in which pleated regions in the package are used to enable a lengthwise extendability of the package when the package is used as a catheter extension during use.

US 4 568 334 and WO 2006/074159 are both related to intravascular catheter assemblies, in which pleated regions in the package are used to facilitate insertion of the catheters into blood vessels.

DE 20 2005 009 946 U1 is related to a urinary catheter assembly in which the catheter is stored in a curved disposition, and in which a urine collection bag is folded over the part of the package housing the catheter.

The described known art, however, requires complicated manufacturing procedures or additional components such as, for instance, a housing, to maintain the catheter in a space-saving controlled condition during storage. Consequently, there is a need for a leaner and less bulky catheter assembly, and/or a catheter assembly which is less expensive to produce, meanwhile easy and convenient to handle and store. Especially there is a need for a catheter assembly which in an improved manner allows the catheter to, during storage, be curved such that minimum space consumption is required, meanwhile kinking of the catheter is avoided.

### Summary of the Invention

It is therefore an object of the present invention to provide a compact catheter assembly of the type mentioned by way of introduction, a method for activating the assembly and a method for manufacturing the same, in which the above-related drawbacks are eliminated wholly or at least partly.

According to a first aspect of the invention, there is provided a catheter assembly according to claim 1.

The invention likewise concerns a method for activation of a catheter assembly of the kind defined above according to claim 27.

Furthermore, the invention likewise concerns a method of manufacturing a catheter assembly of the kind defined above. Consequently, according to another aspect of the invention, there is provided a method of manufacturing a catheter assembly according to claim 28.

Similar to the pleated regions of the present invention, US 5,409,469 discloses an introducer system comprising a sheath having a lumen configured to permit introduction of a lead there through, which sheath has a flexible kink resistant section comprising for instance a series of bellows. The described sheath is, however, applicable for intravenous insertion in a (human) body, and is thus inserted into veins such as the subclavian vein. The described sheath is consequently not intended for storage of a catheter prior to use, the range of use thus being totally different from that of the tubular member of the present invention.

By "storage state" is in this application meant any period of time in which the catheter assembly is stored before use. Preferably, the assembly is arranged in the compact storage state during manufacturing of the assembly, and maintained in this compact state until it is prepared for use. However, the user may also bring the assembly into a straight, non-compact disposition a certain time before use. Still further, the assembly may also be manufactured and sold in a straight disposition, and then be arranged into the compact storage state by the user, e.g. when the catheter assembly is to be brought along in a handbag, a pocket or the like. Thus, "storage state" is not necessarily to be construed to mean the entire time between manufacture and intended use, but may also encompass only a certain part of this time.

A compact catheter assembly in accordance with the present invention may, on the other hand, in a storage state be arranged in a advantageous manner, with the plurality of pleats of the at least one pleated region of the tubular member enabling curved dispositions of catheter sections located therein, and thereby storage of the catheter assembly in a space efficient manner. In addition to enabling for efficient storing of the catheter assembly, the tubular member with its pleated regions, which may resemble a straw with curved regions, also enables for the part of the catheter located therein to in a storage state be arranged such that formation of kinks and distortion is avoided. The plurality of pleats of the at least one pleated region are thus in a storage state arranged with curvatures ensuring that catheter sections located therein are not exposed to curvature to a degree which imposes risking formation of kinks. The controlled curvatures of the pleated regions are consequently adapted to preventing difficulties with straightening of the catheter prior to an intended use, which due to shape memory characteristics may pose a problem when storing the catheter in a space efficient manner for a long period of time, e.g. three to four years. Although the entire insertable length of the catheter preferably is accommodated by the tubular member, it is also feasible that the tubular member accommodates only a catheter section including the insertion end, but where part of the insertable length resides outside that tubular member. Since at least the insertion end of the catheter may be arranged in a straight disposition, the curved sections being arranged further down the catheter, at least the discomfort a user may experience due to a non-straight disposition of the critical first section of the insertable part of the catheter, may be avoided.

In order to allow a pleated region to in a storage state remain in its given curved disposition, the material forming the tubular member is preferably, although not restricted thereto, a rigid, flexible plastics material. With the provision of a tubular member of a rigid material, a given disposition of the tubular member and subsequently the part of the catheter it accommodates, may thus be maintained until an intended use of the catheter. A suitable curvature of a pleated region depends on factors such as the material of the catheter, the diameter of the catheter etc., and is preferably adapted to the specific catheter assembly at hand.

Furthermore, in order to enable the curvature of a pleated region, the pleats forming the pleated region each preferably stretches all the way around a periphery of the tubular member, but a pleat may likewise stretch along only part of the periphery of the tubular member, preferably on the inside of the curvature.

With the number of pleats and their design, and the choice of material, a pleated region may thus be restricted to form a certain curvature. To even further ensure avoidance of kinking and distortion of the catheter, a pleated region is preferably adapted to maintain a minimum diameter curve for the catheter in the storage state.

A pleated region of the present invention preferably forms a bellow-like section of the tubular member. Thereby, the pleated region arranged with a plurality of pleats, is easily arranged to obtain the desired curvature of the storage state. Likewise, as the tubular member is preferably adapted to be straightened to an uncurved disposition prior to an intended use, the bellow-like section preferably contributes to easy straightening of the pleated region, and subsequently straightening of the catheter section located therein, to an uncurved disposition, and to keep the tubular member in any formed disposition, such as the straight or curved disposition.

The catheter assembly preferably comprises at least two and preferably three separated pleated regions, whereby the catheter assembly in the storage state, may be arranged with several curved sections of the catheter. In the case of two separated pleated regions, one region is preferably arranged along the tubular member close to the end accommodating the discharge end of the catheter, and the other region somewhere between the end accomodating the insertable end of the catheter and the point of the tubular member corresponding to the midpoint of the insertable length of the catheter. The invention may however likewise comprise only one, or alternatively even three or more, separated pleated regions. To even further enabling storing of the catheter assembly in a space efficient manner, a maximum length of the catheter assembly is, in the storage state, preferably less than half a length of the catheter, and preferably less than a third of the catheter length. Since especially catheters for male users need to be of a sufficient length, and thus are especially space consuming, the present invention with is ability to efficiently provide space efficient storage, is naturally particularly suitable for these catheters. Thus the insertable length of a catheter in accordance with the present invention preferably exceeds 200 mm, and even more preferably 300 mm.

In order to provide a narrow surrounding for the catheter, the tubular member preferably has an internal diameter only slightly larger than an outer diameter of the catheter. The term "narrow surrounding" is, in the context of this application, to be understood in a broad sense, meaning that the diameter of the tubular member essentially matches that of the catheter, with a difference between the two sufficient enough to enable fluid to surround the catheter meanwhile not being excessive. The diameter difference may for instance be in the range 0.5 to 10 mm, more preferred in the range 0.5 to 5 mm, and most preferred in the range 0.5 to 2 mm.

The catheter is a urinary catheter, and preferably intended for intermittent use. In order to facilitate insertion into a human cavity, the catheter has, on at least part of its surface, a hydrophilic surface layer providing low-friction surface character of the catheter by treatment with a wetting fluid. The catheter assembly in accordance with the present invention also preferably comprises a wetting fluid. The wetting fluid may be arranged in constant wetting contact with the hydrophilic surface layer, for preservation of the hydrophilic surface layer in a wetted state during accommodation in the tubular member and provision of a ready-to-use catheter assembly. Alternatively, the wetting fluid may be arranged in a separate wetting fluid compartment, said compartment being integrated with or arranged in the receptacle. Thus, the compartment keeps the wetting fluid separated from the hydrophilic surface layer of the catheter during accommodation in the tubular member, and the wetting fluid is releasable in an activation state to be brought into contact with the hydrophilic surface layer prior to intended use.

Preferably, at least part of the receptacle forms a pouch accommodating the discharge end of the catheter, the pouch being in fluid communication with the tubular member. Thereby the hydrophilic surface layer of the catheter, if a layer is provided, may be wetted by fluid released in the pouch prior to an intended use. The catheter assembly furthermore preferably comprises a compartment, such as a sachet, containing the wetting fluid, the sachet being accommodated in the pouch, whereby the wetting fluid is kept separated from the hydrophilic surface layer of the catheter during accommodation in the tubular member. The compartment is further preferably arranged, in the activation state, to overlap the discharge end of the catheter and to be opened in an end facing the tubular member. The compartment is thereby arranged to in an activation state be opened, e.g. by compressing the compartment, in an end adjacent to the connection to the tubular member meanwhile remote from the outlet of the discharge end. Wetting fluid being brought into contact with the discharge end is thus avoided. In order to even further eliminate the risk of wetting fluid entering the discharge end, the pouch may additionally be arranged to, in the activation state, keep the discharge end of the catheter separated from the wetting fluid by means of a separation wall.

With the intention of facilitating for the wetting fluid to be transferred from the pouch to the tubular member, the pouch is preferably inwardly tapered towards the connection of the tubular member.

Furthermore, the pouch preferably comprises at least one opening for withdrawal of the catheter prior to intended use. The opening may be a re-sealable opening, and preferably a peel opening. The term "re-sealable opening" is, in the context of this application, to be understood in a broad sense, meaning an opening which may be opened and closed a plurality of times. Furthermore, the term "peel opening" is, in the context of this application, likewise to be understood in a broad sense, meaning an opening formed by e.g. parts of the receptacle material, which is kept closed by, for instance, means of adhesive, which opening may be peeled open. Alternatively, the opening may likewise be a tear opening. The term "tear opening" is, in the context of this application, too to be understood in a broad sense, meaning an opening which is torn open by pulling parts of the receptacle material in essentially opposite directions.

In accordance with the present invention, the tubular member preferably comprises a detachable cover at the end housing the insertion end of the catheter, the remaining tubular member after detachment being adapted to be attached to the discharge end of the catheter. Furthermore, the discharge end of the catheter is preferably a connector. The term "connector" is, in the context of this application, to be understood in a broad sense, meaning a part of the catheter with a flared end functioning as a means for connecting the catheter tube to external means, such as external tubing, or simply functioning as an outlet for drainage through the catheter tube. Further, the connector need not be a separate part of the catheter, but could be integrated with the catheter tube. Preferably, the remaining tubular member is furthermore arranged to be attached to the connector. Preferably, the end of the tubular member is frustoconical, corresponding to the flared end of the catheter, thereby being suitably connectable by insertion into said connector. Connector or not, either or both ends of the remaining tubular member is preferably adapted for attachment to the discharge end, and attachment may for instance be accomplished by means of the mating surfaces having essentially matching shapes. Thereby, after attachment of the remaining tubular member to the discharge end, friction will secure them to one another until an intended detachment. The present invention is however not restricted to attachments based on friction, the remaining tubular member and the discharge end of the catheter may likewise be attached to one another by any appropriate means, for instance by snap fitting. In providing the detachable cover and additionally a remaining tubular member which in one of its ends is adapted to be attached to the discharge end of the catheter, a guiding tube extension is provided, which during use of the catheter may facilitate drainage of for instance urine, into for instance a water closet. In addition to provision of an extended drainage aid, the pleated regions of the guiding tube extension, which preferably as previously mentioned is of a rigid material, furthermore enable for the user of the catheter, or caretaker, to arrange the extension in a manner appropriate for the present circumstances. That is, the drainage aid may be formed, and advantageously maintained, in a disposition adapted for drainage into the drainage collection means, e.g. a bowl, for improved guiding according to the present conditions, and to remain safely in place during drainage.

The catheter assembly of the present invention furthermore preferably comprises a tubular insertion aid arranged at least partly around at least part of the discharge end of the catheter, wherein the tubular insertion aid is arranged to, after withdrawal of the catheter from the receptacle, being movable along the catheter to thereby function as an insertion aid for maneuvring the wetted catheter during insertion into the human cavity. The tubular insertion aid is preferably initially arranged around the connector should one be comprised in the catheter assembly, and thus preferably has a shape narrowly surrounding the connector. Thus the shape of the tubular insertion aid preferably essentially matches that of the discharge end, with a difference between the two sufficient enough to enable separation of the two meanwhile not being excessive. Designing the tubular insertion aid such that it may be arranged around the discharge end / connector enables for the tubular insertion aid to be moved along the catheter during insertion, thereby preventing the need to directly touch the catheter surface. Thus, provision of the tubular insertion aid in this way does not affect the length of the catheter assembly, i.e. the catheter and receptacle do not need to be designed to have an extended length in order to make room for the tubular insertion aid. Furthermore, in order to enable the tubular insertion aid to be compressed, and thereby holding and controlling of the catheter in a "no touch"-manner using the tubular insertion aid, the tubular insertion aid is preferably, although not necessarily, made of a flexible material. In providing a tubular insertion aid which enables maneuvering of the catheter during and after use without having to touch the actual catheter, the risk of contamination is reduced.

The tubular insertion aid has furthermore preferably a slit along a longitudinal direction. The tubular insertion aid is thereby easily detachable from the catheter, as the slit may be utilized for removal of the tubular insertion aid from the catheter. The possibility of removal of the tubular insertion aid enables, in the same manner as described in conjunction with the tubular insertion aid having a shape surrounding the connector / discharge end, for the length of the catheter to be unaffected by the presence of the tubular insertion aid. That is, even if essentially the entire insertable length of the catheter should be required for insertion during use, the catheter does not need to be designed having an extended length in order to make room for the tubular insertion aid. Instead , the insertion aid can easily be released from the catheter shaft when insertion is completed or nearly completed.

The receptacle may form a urine collection bag.

Additionally, the catheter assembly furthermore comprises an outer case enclosing the catheter assembly, the outer case being arranged to maintain the receptacle in the curved disposition of the storage state. The outer case, which may be of any material and shape appropriate, and which preferably, although not necessarily, is re-sealable, thus enables for provision of a lean catheter assembly, easily handled and stored. For instance, the outer case may be in the form of a sleeve surrounding the catheter assembly. The sleeve may be of plastic or paper material.

A catheter assembly in accordance with the present invention may, after use, be reverted to its storage state. That is, the catheter may after use be re-inserted into the receptacle, and the used catheter assembly in turn be re-placed in the outer case, should one be provided. The catheter assembly may thus after use be reverted to a compact package, thereby facilitating disposal of the assembly. This possibility enables the user to handle the assembly with more discretion also after the catheter has been used, and may even allow the user to put the used assembly in his/her pocket for later disposal.

As have been described in the foregoing, a catheter assembly in accordance with the present invention is cost effective to produce, meanwhile easy and convenient to handle and store. It is compact and possible to store discreetly in for instance the pocket of a users clothing. In an improved manner, the catheter assembly allows the catheter to, during storage, be curved such that minimum space consumption is required, and at the same time kinking of the catheter is avoided.

Other aspects, benefits and advantageous features of the invention will be apparent from the following description and claims.

### Brief Description of the Drawings

The invention will be more apparent from the accompanying drawings, which are provided by way of non-limiting examples.
Figure 1 shows an exemplary catheter for use in catheter assemblies of the present invention.
Figure 2 is an illustration of a catheter assembly in a storage state in accordance with an exemplary embodiment of the present inventive concept.
Figure 3a and 3b show the catheter assembly of Figure 2 comprised in two different emobiments of outer case maintaining the receptacle in the curved disposition of the storage state.
Figure 4 illustrates the catheter assembly of Figure 2 with the tubular member in an uncurved disposition.
Figure 5 shows the catheter assembly of Figure 2 with the tubular member in an uncurved disposition, with the catheter partly withdrawn.
Figure 6 illustrates a fully withdrawn catheter, onto which discharge end, the remaining tubular member in accordance with another exemplifying embodiment, may be attached.
Figure 7 is a flowchart showing exemplary steps for activation of the catheter assembly of Figure 6.
Figure 8 is a flowchart describing exemplifying steps to manufacture a catheter assembly in accordance with the embodiment shown in Figure 2.

### Detailed Description of Preferred Embodiments of the Invention

In the following detailed description, preferred embodiments of the present invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, for instance the length of the catheter and dimensions of parts comprised in the catheter assembly etc.

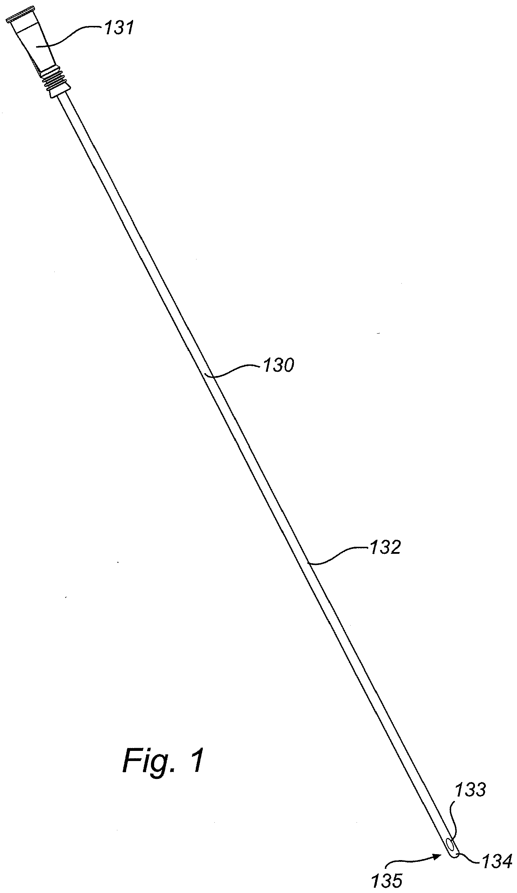

In Figure 1, a known urinary catheter 130 intended for intermittent use, is illustrated. The catheter 130 comprises a flared discharge end 131 and an elongate shaft or tube 132 projecting from the discharge end 131 to an insertion end 135. An open-ended internal lumen (not shown) extends from the discharge end 131 to a drainage aperture 133 in a rounded tip 134 in the insertion end 135 of the elongate tube 132. The discharge end 131 may function as a connector of the catheter 130, being connectable to other devices, such as a urine collection bag, a drainage tube or the like.

At least a part of the elongate tube 132 forms an insertable length to be inserted through a body opening of the user, such as the urethra in case of a urinary catheter. By "insertable length" is normally, in the context of a hydrophilic catheter, meant the length of the elongate tube 132 which is coated with a hydrophilic material, for example PVP, and which is insertable into the urethra of the patient. Typically, this will be 80-140 mm for a female patient and 200-350 mm for a male patient.

Many different types of well-known hydrophilic surfaces may be used. For example, the catheter may be provided with a hydrophilic coating wherein the hydrophilic polymer coating comprises material selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. The preferred hydrophilic polymer is polyvinylpyrrolidone.

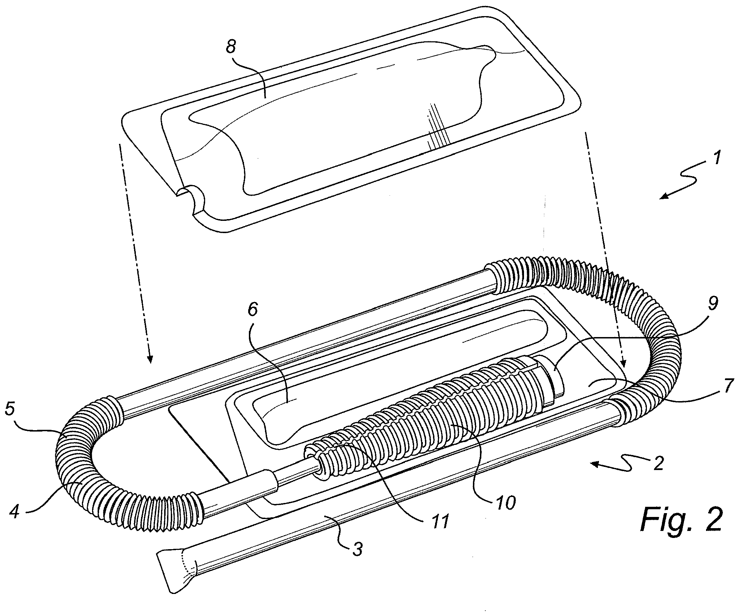

In a storage state, i.e. prior to use, the catheter 130 is accommodated by a receptacle which provides sterile conditions.. In Figure 2 is illustrated in a partly exploded view, a catheter assembly 1 in accordance with an embodiment, comprising a receptacle 2 accommodating the catheter 130. The receptacle 2 may (not shown) form a urine collection bag.

The receptacle 2 furthermore comprises a tubular member 3, which accommodates at least the insertion end 135 of the catheter 130. The material forming the tubular member 3 is preferably although not restricted thereto a rigid, flexible plastics material, furthermore preferably more rigid than the flexible plastics material potentially forming other parts of the receptacle 2. The pleated regions 4 maintain the tubular member for instance in a curved disposition. In providing a tubular member 3 of a rigid material, a curved disposition of the tubular member 3 and subsequently the part of the catheter 130 it accommodates, may thus be maintained.

Although in the illustrated embodiment the entire elongate tube 132 is accommodated by the tubular member 3, the invention is not restricted thereto, the tubular member 3 may likewise accommodate only part of the catheter 130 including the insertion end 135. The insertable length of the catheter 130 preferably exceeds 200 mm, and even more preferably 300 mm. The catheter assembly 1 is preferably for male catheters 130.

The tubular member 3 has at least one pleated region 4 located along the tubular member 3, arranged with a plurality of pleats 5. The pleats 5 each preferably extends all the way around a periphery of the tubular member 3, as shown in Figure 2, but a pleat may likewise extend along only part of the periphery of the tubular member 3. In the storage state, the pleats 5 forms a curvature, whereby a catheter section located therein is arranged in a curved disposition. In the exemplary embodiment of Figure 2, the tubular member 3 comprises two separated pleated regions 4 forming bellow-like sections, which enables the catheter assembly 1 to, in a storage state, be curved such that a length of the catheter assembly 1 is less than half the length of the catheter 130, and preferably about a third of said length. The invention may however likewise comprise only one, or three or more separated pleated regions 4. The pleated regions 4 are adapted to maintain a minimum diameter curve for the catheter 130 in the storage state, such that kinking and distortion of the catheter 130 is avoided. In this manner, difficulties with reverting the catheter 130 to its straight disposition prior to an intended use, may be avoided.

In order to avoid exposing the user of the catheter 130 for discomfort, a straight disposition of the catheter 130 during use is specifically critical for the first part being inserted into the human cavity, i.e. the insertion end 131. Consequently, approximately the first 100 mm of the insertion end 135 of the catheter 130 are preferably constantly maintained in an uncurved disposition, thereby even further contributing to avoiding discomfort for the user during insertion. As shown in Figure 2, a corresponding length of the end of the tubular member 3 housing the insertion end 131 of the catheter 130 does therefore preferably not comprise any pleated regions 4.

The tubular member 3 furthermore has an internal diameter which is preferably only slightly larger than an outer diameter of the catheter 130, whereby the tubular member 3 provides a narrow surrounding for the catheter 130.

In the exemplary embodiment of Figure 2, the catheter 130 has on at least the insertable length, a hydrophilic surface layer providing low-friction surface character of the catheter 130 by treatment with a wetting fluid. Further, a compartment 6, such as a sachet, comprising a wetting fluid is preferably arranged in the receptacle 2, whereby the wetting fluid is kept separated from the hydrophilic surface layer of the catheter 130 during accommodation in the tubular member 3.

Furthermore, a part of the receptacle 2 in the exemplary embodiment shown forms a pouch 7, which accommodates the discharge end 131 of the catheter 130 as well as the compartment 6. The pouch 7 is in fluid communication with the tubular member 3, such that the hydrophilic surface layer of the catheter 130, if a layer is provided, may be wetted by fluid released in the pouch 7 prior to an intended use. The compartment 6 is here arranged to overlap the discharge end 131 of the catheter 130 and be opened in an end facing the tubular member 3. Wetting fluid being brought into contact with the outlet of the discharge end 131 is thus avoided. The pouch 7 may furthermore, although not shown, be arranged to keep the discharge end 131 of the catheter 130 separated from the wetting fluid by means of a separation wall. The separation wall is preferably part of the pouch 7. Additionally, and likewise not shown, the pouch 7 may be inwardly tapered towards the connection of the tubular member 3, whereby transfer of the wetting fluid from the pouch 7 to the tubular member 3 in an activation state, is facilitated.

Preferably, the pouch 7 comprises at least one opening 8 in order to expose the catheter 130 for withdrawal prior to an intended use, and preferably a re-sealable peel opening 8. However, the pouch 7 may comprise any number of openings 8, which may be of any sort, such as for instance tear openings.

Although a catheter assembly 1 comprising a catheter 130 having a hydrophilic surface layer, a part of the receptacle 2 forming a pouch 7 and a compartment 6 comprising the wetting fluid is preferred, the present invention is not restricted thereto. The wetting fluid may for instance be arranged in a wetting fluid compartment of the receptacle 2 or even the pouch 7, separating the wetting fluid from the catheter 130 during accommodation in the tubular member 3, thereby eliminating the need for a separate compartment 6. Additionally, for instance when wetting fluid is not provided with the catheter assembly 1, the pouch 7 may be superfluous. Alternatively, the wetting fluid may be arranged in wetting contact with the hydrophilic layer, for preservation of the hydrophilic surface layer in a wetted state during accommodation in the tubular member 3, and thereby provision of a ready-to-use catheter assembly 1.

Preferably, the catheter assembly 1 furthermore comprises a tubular insertion aid 10 arranged at least partly around at least part of the discharge end or connector 131 of the catheter 130. The tubular insertion aid 10 is during and after withdrawal of the catheter 130 from the receptacle 2 movable along the catheter 130 to thereby function as an insertion aid for maneuvering the wetted catheter 130 for instance during insertion into the human cavity. The tubular insertion aid 10 is preferably initially arranged around the discharge end / connector, and thus preferably, and as shown in Figure 2, has a shape narrowly surrounding the connector. Designing the tubular insertion aid 10 such that it may be arranged around the connector 9 enables for a very compact catheter assembly 1 and also enables for the user of the catheter 130, or caretaker, to during insertion into the human cavity, if necessary due to the insertion requiring essentially the entire insertable length of the catheter 130, move the tubular insertion aid 10 all the way back to surrounding the connector 19. Provision of the tubular insertion aid 10 does consequently not affect the length of the catheter 130, i.e. the catheter 130 does not need to be designed to have an extended length in order to make room for the tubular insertion aid 10 when the cathter is inserted into the urethra.

The flexible material of which the tubular insertion aid 10 is preferably made, most preferably flexible plastics material, enables the tubular insertion aid 10 to be compressed, and the user of the catheter 130, or caretaker, to thereby guide and hold the catheter 130 in a "no touch"-manner using the tubular insertion aid 10. The tubular insertion aid 10 subsequently enables a user of the catheter 130, or a caretaker, to maneuver the catheter 130 during and after use without having to touch the actual catheter 130, whereby the risk of contamination is reduced.

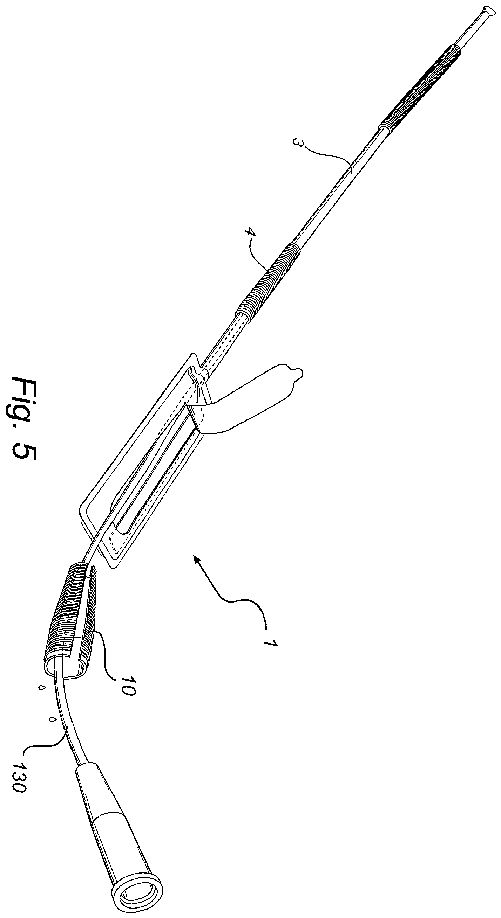

The tubular insertion aid 10 has furthermore preferably, and as shown in the embodiment of Figure 2, a slit 11 along a longitudinal direction. The tubular insertion aid 10 is thereby easily detachable form the catheter 130, as the slit 11 may be utilized for removal of the tubular insertion aid 10 from the catheter 130 during or after use. The width of the slit 11 is preferably smaller than an outer diameter of the catheter 130 (best illustrated in Figure 5). With this dimension of the slit 11 in relation to the catheter 130, removal of the tubular insertion aid 10 from the catheter 130 should be fairly simple meanwhile there should be no risk of the tubular insertion aid 10 being detached from the catheter 130 unintentionally. Notably, the insertion aid could be used in various ways by different users, and could e.g. be removed before insertion into the urethra, during the insertion, after the insertion is completed or even not be removed at all.

The possibility of removal of the tubular insertion aid 10 furthermore enables for the length of the catheter 130 to be unaffected by the presence of the tubular insertion aid 10. That is, even if essentially the entire insertable length of the catheter 130 should be required for insertion during use, the catheter 130 does not need to be designed having extended length in order to make room for the tubular insertion aid 10.

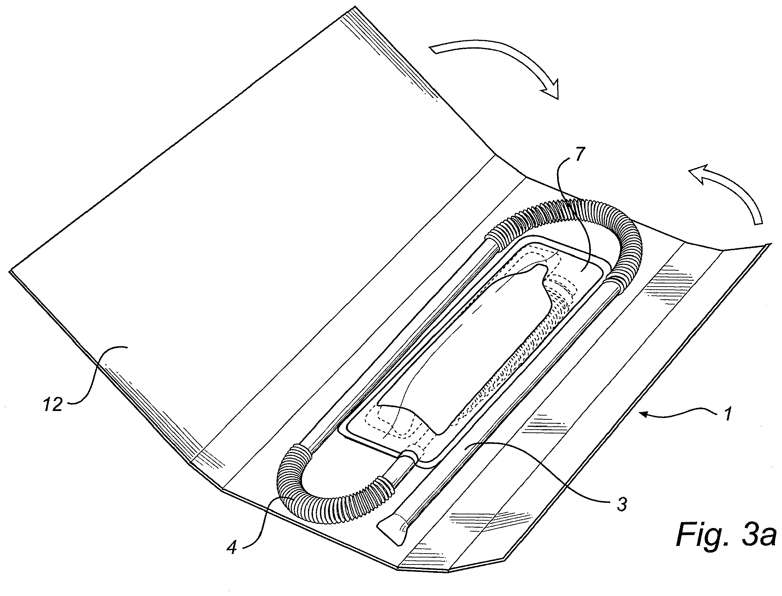

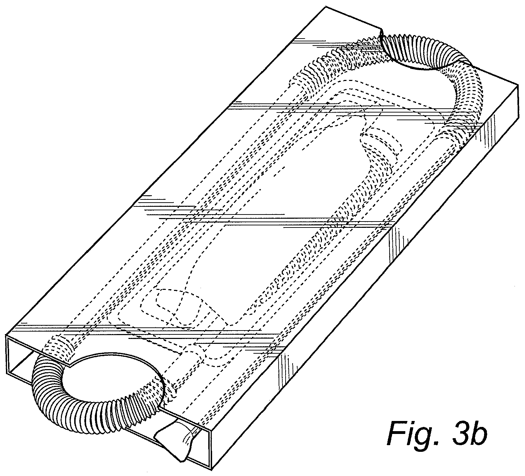

The catheter assembly 1 of the embodiment described above, as is shown in Figures 3a and 3b, furthermore comprises an outer case 12 enclosing the catheter assembly 1. The outer case 12 is arranged to maintain the receptacle in the curved disposition of the storage state and to form an extra protective cover. However, as discussed above the tubular member in itself maintains the catheter assembly in any desired disposition, and the outer case is therefore not needed to maintain the curved disposition, but adds an extra measure to maintain this disposition during storage. Further, the outer case also makes the catheter product more discret, and makes the nature of the product less discernible for anyone seeing the product. Thus, the outer case enhances the privacy for the user. Each of the open ends of the outer case 12 enables for withdrawal of the receptacle 2 prior to an intended use, and removal of the receptacle 2 from the outer case 12, as is seen in the embodiment of Fig 3b. However, in the embodiment of fig 3a, removal is even further facilitated in that the outer case 12 is also openable as well as sealable. The shown outer case 12 is however merely exemplary, and other variants of outer cases 12, such as for instance boxes, are likewise possible within the scope of the invention.

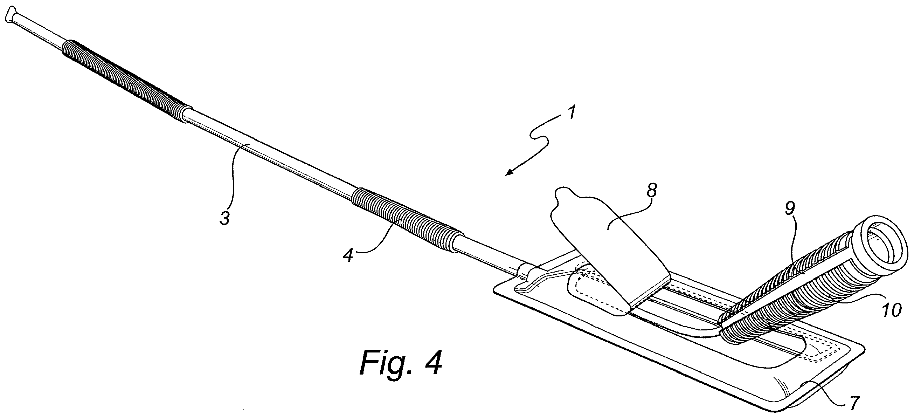

Prior to an intended use, the catheter 130 will need to leave its storage state and eventually enter an activation state. Figure 4 thus illustrates the catheter assembly 1 of the above-described embodiment with the tubular member 3 in an uncurved disposition. The bellow-like pleated regions 4 have been straightened out such that the catheter sections located therein and the pleated regions 4 adopt substantially uncurved dispositions. Subsequently, the re-sealable peel opening 8 of the pouch 7 has been opened to enable withdrawal of the catheter 130. The connector 9 is here shown as being fully withdrawn, whereas the rest of the catheter 130 along with the tubular insertion aid 10 still remains in the tubular member 3.

In Figure 5, the described embodiment of the present invention is illustrated with the catheter assembly 1 in an uncurved disposition, with the catheter shaft now partly withdrawn. The preferred tubular insertion aid 10 is here fully withdrawn, and as previously described, movable along the catheter 130 for manoeuvring of the catheter 130. Before withdrawal, the hydrophilic layer of the catheter 130 of the exemplary embodiment has been wetted.

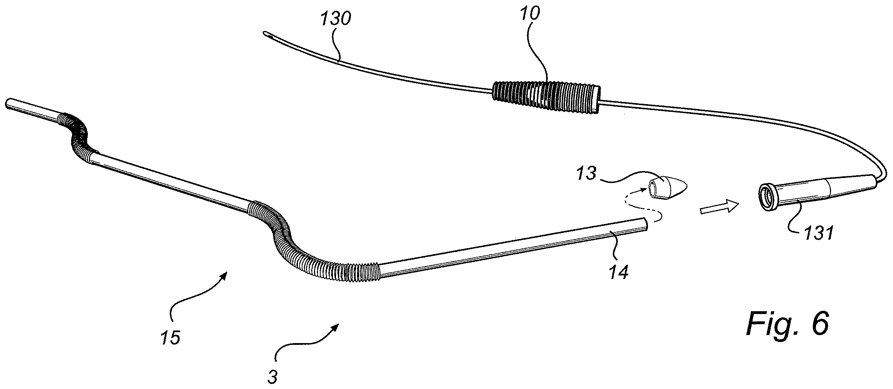

In another exemplifying embodiment of the present invention, shown in Figure 6, the tubular member 3 furthermore comprises a detachable cover 13 at the end housing the insertion end 135 of the catheter 130. In Figure 6, the detachable cover 13 has been removed from the insertion end 135 of the catheter 131 along with the remaining tubular member 14. The remaining tubular member 14 is in one of its ends adapted to be attached to the discharge end 131 of the catheter 130. Note however, that either or both ends of the remaining tubular member 14 may be adapted for attachment to the discharge end 131. Preferably, the remaining tubular member 14 is attachable to the discharge end 131, here the connector 9, in that the mating surfaces have essentially matching shapes. Thereby, after attachment of the remaining tubular member 14 to the discharge end 131, friction will secure them to one another until an intended detachment. However, alternatively the remaining tubular member 14 and the discharge end 131 of the catheter 130 may likewise be attached to one another by any appropriate means, for instance by snap fitting. In providing the detachable cover 13 and additionally the remaining tubular member 14 which in one of its ends is adapted to be attached to the discharge end 131 of the catheter 130, a guiding tube extension 15 is provided, which during use of the catheter 130 may facilitate drainage of for instance urine, into e.g. a bowl. In addition to provision of an extended drainage aid 15, the pleated regions 4 of the guiding tube extension 15 furthermore enable for the user of the catheter 130, or caretaker, to arrange the extension 15 in a manner appropriate for the present circumstances. That is, the drainage aid 15 may be formed, and advantageously maintained, in a disposition adapted for drainage into the drainage collection means, e.g. a bowl, for improved guiding according to the present conditions.

Further, it is also possible to use the tubular member as an extension connectable to the discharge end of the catheter without separating the tubular member from the pouch. In such an embodiment, the discharge urine may instead be lead trough the pouch, to a discharge opening in said pouch, the pouch may be turn inside out over the discharge end of the tubular member, or the like.

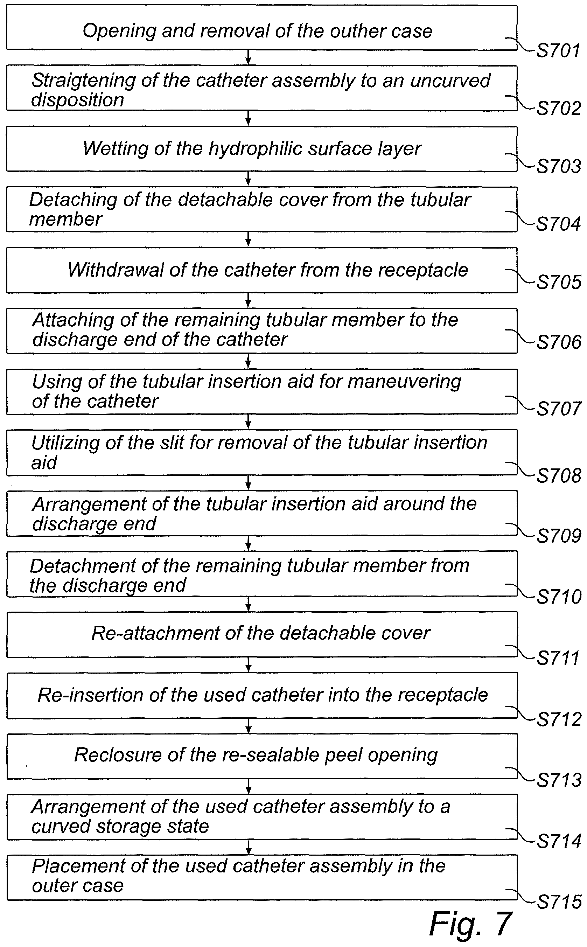

In the following, a flowchart showing exemplary steps for activation of the catheter assembly 1 of Figure 6, is illustrated in Figure 7.

First, in step S701, the outer case 12 enclosing the catheter assembly 1, is opened and removed. As previously mentioned, the outer case 12 may be of various types and shapes, whereby merely withdrawal of the catheter assembly 1, without opening of the outer case 12, may thus be sufficient for some cases.

Next, in step S702, the catheter assembly 1 is straightened to an uncurved disposition, and in step S703, the hydrophilic layer of the catheter 130 is wetted. Wetting of the catheter may be achieved in various manners, and in accordance with the preferred embodiment described in Figures 2 to 5 and 6, the activation is performed by pressing, tearing, piercing, or twisting, etc, which is per se well known in the art, of the compartment 6, and releasing of the wetting fluid into the pouch 7. Thereby, the wetting fluid is, in that the catheter assembly is held in an essentially vertical position with the pouch 7 in a higher position than that of the tubular member 3, enabled to flow down into the tubular member 3. With the provision of the wetting fluid into the tubular member 3, the wetting fluid is brought into contact with the hydrophilic coating of the catheter 130.

After a sufficient wetting period, the preferred detachable cover 13 of the tubular member 3 is, in step S704, detached from the remaining tubular member 14.

Next, in step S705, the catheter 130 is withdrawn from the receptacle 2. In the preferred embodiment in accordance with Figure 2 to 5 and 6, that implies withdrawal of the catheter 130 from the remaining tubular member 14 and the pouch 7, through the re-sealable peel opening 8. The detachable cover 13, the compartment 6 and/or the pouch 7 may be disposed of.

After withdrawal of the catheter 130 in step S706, the remaining tubular member 14 is, with one of its ends, attached to the discharge end 131 of the catheter 130, thereby forming a guiding tube extension 15.

In order to maneuver the catheter 130, the tubular insertion aid 10 is preferably, in step S707, moved from its original position around the discharge end 131 of the catheter 130, and then maneuvered along the catheter 130 as found convenient or necessary. The tubular insertion aid 10 may then in step S708, be removed from the catheter 130. Preferably, and as previously described, the tubular insertion aid 10 has a slit 11 along a longitudinal direction, which is utilized to facilitate the detaching of the tubular insertion aid 10.

During use of the catheter, the guiding tube extension 15 is preferably used to guide for instance the urine into e.g. a toilet or bowl as previously described.

The remaining parts of the used catheter assembly 1 may be disposed of. Should however lack of an available waste container, or discretion, render immediate disposal of the used catheter assembly 1 impossible, the embodiments of Figures 2 and 6 in accordance with the present invention furthermore support reverting the used catheter assembly 1 to a storage state, to thereby facilitate later disposal of the assembly 1.

Thus, in accordance with step S709, the tubular insertion aid 10 may be brought back along the catheter 130 to be arranged around the discharge end 131.

Next, in step S710, the remaining tubular member 14 may be detached from the discharge end 131, and in step S711, the detachable cover 13 reattached to the remaining tubular member 14.

Then, in step S712, the used catheter 130 may be re-inserted into the receptacle 2, and in step S713, the re-sealable peel opening 8 of the pouch 7 may be reclosed.

The used catheter assembly 1 may then, in step S714, be arranged to its original curved disposition of the storage state.

Ultimately, the used catheter assembly 1 is in step S715 placed in the outer case 12 enclosing the catheter assembly 1, whereby the used catheter assembly may be disposed, carried along or be stowed in a convenient manner.

It should be noted that the order of the steps may be shifted to suit the preferences of the user of the catheter 130, or caretaker. For instance, steps S704 and S705 be switched, and step S707 may be performed repeatedly and simultaneously with the other steps.

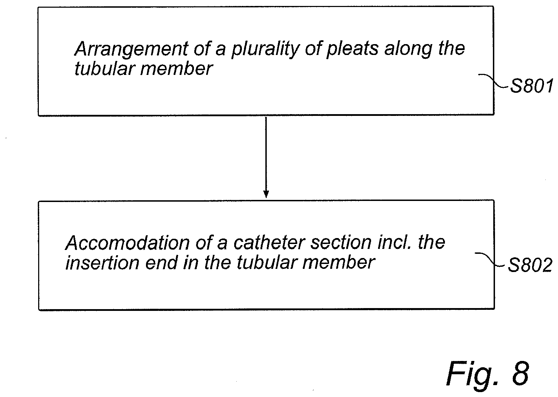

In order to in an exemplifying manner illustrate a procedure of manufacturing an exemplary catheter assembly 1, Figure 8 reveals steps for manufacturing the embodiment of Figure 2.

In a first step, step S801, a plurality of pleats 5 are arranged in at least one pleated region 4 along the tubular member 3. Then, in step S802, a catheter section of the described preferred catheter 130 of Figure 2 including the insertion end 135 of the catheter 130 is accommodated in the tubular member 3. Thereafter, the pleats 5 are curved to a curved disposition together with the catheter section located therein and arranged in outer case 12. Thereafter, the whole assembly may be sterilized by radiation.

It should be noted that the order of the steps in Figure 8 may be shifted.

The invention has now been discussed in relation to different embodiments. However, it should be appreciated by those skilled in the art that several further alternatives are possible. For example, the features of the different embodiments discussed above may naturally be combined in many other ways.

Still further, it is possible to arrange the wetting fluid compartment in many different ways. For example, the compartment may be a separate sachet or the like, or forming part of the assembly. The wetting fluid compartment may be arranged completely inside the receptacle, partly inside the receptacle, or completely outside the receptacle. Alternatively, the wetting fluid compartment may be an integrated compartment of the receptacle. Furthermore, the separation wall could e.g. be a breakable or peelable membrane wall, but alternative embodiments are naturally feasible, such as various types of detachable or openable caps or closings.

Furthermore, many different materials could also be used for the different parts of the catheter assembly.
It will be appreciated by those skilled in the art that several such alternatives similar to those described above could be used, and all such modifications should be regarded as a part of the present invention, as defined in the appended claims.

## Claims

1. A catheter assembly (1) comprising a urinary catheter (130) having an insertion end (135) and a discharge end (131), said catheter (130) for at least said insertable length, has on at least part of its surface a hydrophilic surface layer providing low-friction surface character of the catheter (130) by treatment with a wetting fluid, and a receptacle (2), said receptacle (2) comprising a tubular member (3) which in a storage state accommodates a catheter section including the insertion end (135) of said catheter (130), said tubular member (3) having at least one pleated region (4) located along said tubular member (3) arranged with a plurality of pleats (5), **characterized in that** said pleats (5) in said storage state form and maintain a curvature, thereby enabling for a curved disposition of a catheter section located therein, and **in that** it further comprises an outer case (12) enclosing said receptacle (2) when the receptacle (2) is arranged in said curved disposition of the storage state.

2. The catheter assembly (1) in accordance with claim 1, wherein said pleated region (4) forms a bellow-like section of said tubular member.

3. The catheter assembly (1) in accordance with claim 1 or 2, wherein said pleated region (4) is adapted to maintain a minimum diameter curve for the catheter (130) in said storage state.

4. The catheter assembly (1) in accordance with any one of the preceding claims, wherein said catheter assembly (1) comprises at least two and preferably three separated pleated regions (4).

5. The catheter assembly (1) in accordance with any one of the preceding claims, wherein said tubular member (3) prior to an intended use, is adapted to be straightened to an uncurved disposition.

6. The catheter assembly (1) in accordance with any one of the preceding claims, wherein in said storage state, a maximum length of said catheter assembly (1) is less than half a length of said catheter (130), and preferably about a third of said catheter length.

7. The catheter assembly (1) in accordance with any one of the preceding claims, wherein the length of said catheter (130) exceeds 200 mm, and preferably exceeds 300 mm.

8. The catheter assembly (1) in accordance with any one of the preceding claims, wherein said tubular member (3) has an internal diameter only slightly larger than an outer diameter of said catheter (130), said tubular member (3) thereby providing a narrow surrounding for said catheter (130).

9. The catheter assembly (1) in accordance with any one of the preceding claims, wherein said catheter (130) is a urinary catheter intended for intermittent use.

10. The catheter assembly (1) in accordance with any one of the preceding claims, further comprising a wetting fluid.

11. The catheter assembly (1) in accordance with claim 10, wherein said wetting fluid is arranged in wetting contact with said hydrophilic surface layer, for preservation of said hydrophilic surface layer in a wetted state during accommodation in said tubular member (3) and provision of a ready-to-use catheter assembly (1).

12. The catheter assembly (1) in accordance with claim 10, wherein said wetting fluid is arranged in a wetting fluid compartment keeping the wetting fluid separated from said hydrophilic surface layer of said catheter (130) during accommodation in said tubular member (3), and said wetting fluid is releasable in an activation state to be brought into contact with said hydrophilic surface layer prior to intended use.

13. The catheter assembly (1) in accordance with any one of the preceding claims, wherein at least part of said receptacle (2) forms a pouch (7) accommodating said discharge end (131) of said catheter (130), said pouch (7) being in fluid communication with said tubular member (3).

14. The catheter assembly (1) in accordance with claim 13 in combination with claim 13, further comprising a compartment (6), such as a sachet, containing said wetting fluid, said compartment (6) being accommodated in said pouch (7).

15. The catheter assembly (1) in accordance with claim 14, wherein said compartment (6) is arranged, in said activation state, to overlap the discharge end (131) of said catheter (130) and be opened in an end facing said tubular member (3).

16. The catheter assembly (1) in accordance with claim 14 or 15, wherein said pouch (7) is arranged to, in said activation state, keep the discharge end (131) of said catheter (130) separated from said wetting fluid by means of a separation wall.

17. The catheter assembly (1) in accordance with any one of claim 13 to 16, wherein said pouch (7) inwardly is tapered towards the connection of said tubular member (3), thereby facilitating for said wetting fluid to be transferred from said pouch (7) to said tubular member (3).

18. The catheter assembly (1) in accordance with any one of claim 13 to 17, wherein said pouch (7) comprises at least one opening (8) for withdrawal of said catheter (130) prior to intended use.

19. The catheter assembly (1) in accordance with claim 18, wherein said opening (8) is a re-sealable opening, and preferably a peel opening.

20. The catheter assembly (1) in accordance with claim 18 or 19, wherein said opening (8) is a tear opening.

21. The catheter assembly (1) in accordance with any one of the preceding claims, wherein said tubular member (3) comprises a detachable cover (13) at the end housing said insertion end (135) of the catheter (130), the remaining tubular member (14) being adapted to be attached to said discharge end (131) of the catheter (130).

22. The catheter assembly (1) in accordance with any one of the preceding claims, wherein the catheter (130) further comprises a connector (9), one end of said connector (9) forming said discharge end (131) of the catheter (130).

23. The catheter assembly (1) in accordance with claim 22 in combination with claim 21, wherein said remaining tubular member (14) is arranged to be attached to said connector (9).

24. The catheter assembly (1) in accordance with any one of the preceding claims, further comprising a tubular insertion aid (10) arranged at least partly around at least part of said discharge end (131) of the catheter (130), wherein said tubular insertion aid (10) is arranged to, after withdrawal of said catheter (130) from said receptacle (2), being movable along said catheter (130) to thereby function as an insertion aid for maneuvering the wetted catheter (130) during insertion into the human cavity.

25. The catheter assembly (1) in accordance with claim 24, wherein said tubular insertion aid (10) has a slit (11) along a longitudinal direction, said tubular insertion aid (10) thereby being easily detachable from said catheter (130) as said slit (11) may be utilized for removal of said tubular insertion aid (10) from said catheter (130).

26. The catheter assembly (1) in accordance with any one of the preceding claims, wherein said receptacle (2) forms a urine collection bag.

27. A method for activation of the catheter assembly in accordance with any one of the preceding claims, comprising:
straightening (S702) said catheter assembly (1) to an uncurved disposition,
wetting (S703) said hydrophilic surface layer, and
withdrawing (S705) said catheter (130) from said receptacle (2).

28. A method of manufacturing a catheter assembly (1), which assembly (1) comprises a urinary catheter (130) having an insertion end (135) and a discharge end (131), and a receptacle (2) comprising a tubular member (3), said method comprising:
arranging (S801) a plurality of pleats (5) in at least one pleated region (4) along said tubular member (3), and
accommodating (S802) a catheter section including the insertion end (135) of said catheter (130) in said tubular member (3),
**characerized in that** said pleats (5) in a storage state form and maintain a curvature, thereby enabling for a curved disposition of a catheter section located therein; and by the further step of enclosing the receptacle (2) in an outer case (12) when the receptacle (2) is arranged in said curved disposition of the storage state.

## Patentansprüche

1. Katheteranordnung (1), die einen Blasenkatheter (130) umfasst, der ein Einführungsende (135) und ein Auslassende (131) hat, wobei der Katheter (130) für mindestens die einführbare Länge auf mindestens einem Teil seiner Oberfläche eine hydrophile Oberflächenschicht hat, die für einen reibungsarmen Oberflächencharakter des Katheters (130) durch Behandlung mit einem Benetzungsfluid sorgt, und eine Aufnahme (2), wobei die Aufnahme (2) ein rohrförmiges Element (3) umfasst, welches im Lagerungszustand einen Katheterabschnitt aufnimmt, einschließlich des Einführungsendes (135) des Katheters (130), wobei das rohrförmige Element (3) mindestens einen gefalteten Bereich (4) hat, der sich entlang des rohrförmigen Elementes (3) befindet, welcher mit einer Vielzahl von Falten (5) angeordnet ist, **dadurch gekennzeichnet, dass** die Falten (5) im Lagerungszustand eine Krümmung bilden und aufrechterhalten, wodurch sie eine gekrümmte Anordnung eines Katheterabschnitts ermöglichen, der sich darin befindet, und dadurch, dass er ferner ein Außengehäuse (12) umfasst, das die Aufnahme (2) umschließt, wenn die Aufnahme (2) in der gekrümmten Anordnung des Lagerungszustands angeordnet ist.

2. Katheteranordnung (1) nach Anspruch 1, wobei die gefaltete Region (4) einen balgartigen Abschnitt des rohrförmigen Elementes bildet.

3. Katheteranordnung (1) nach Anspruch 1 oder 2, wobei die gefaltete Region (4) dafür ausgelegt ist, eine Krümmung mit minimalem Durchmesser für den Katheter (130) im Lagerzustand aufrechtzuerhalten.

4. Katheteranordnung (1) nach einem der vorherigen Ansprüche, wobei die Katheteranordnung (1) mindestens zwei und vorzugsweise drei getrennte gefaltete Regionen (4) umfasst.

5. Katheteranordnung (1) nach einem der vorherigen Ansprüche, wobei das rohrförmige Element (3) vor einer vorgesehenen Verwendung dafür ausgelegt ist, in eine ungekrümmte Anordnung gestreckt zu werden.

6. Katheteranordnung (1) nach einem der vorherigen Ansprüche, wobei im Lagerzustand eine maximale Länge der Katheteranordnung (1) kleiner als eine halbe Länge des Katheters (130) ist und vorzugsweise etwa ein Drittel der Katheterlänge ist.

7. Katheteranordnung (1) nach einem der vorherigen Ansprüche, wobei die Länge des Katheters (130) 200 mm übersteigt und vorzugsweise 300 mm übersteigt.

8. Katheteranordnung (1) nach einem der vorherigen Ansprüche, wobei das rohrförmige Element (3) einen Innendurchmesser hat, der nur leicht größer als ein Außendurchmesser des Katheters (130) ist, wodurch das rohrförmige Element (3) für eine enge Umschließung des Katheters (130) sorgt.

9. Katheteranordnung (1) nach einem der vorherigen Ansprüche, wobei der Katheter (130) ein Blasenkatheter ist, der für den intermittierenden Gebrauch vorgesehen ist.

10. Katheteranordnung (1) nach einem der vorherigen Ansprüche, die ferner ein Benetzungsfluid umfasst.

11. Katheteranordnung (1) nach Anspruch 10, wobei das Benetzungsfluid in benetzendem Kontakt mit der hydrophilen Oberflächenschicht angeordnet ist, zur Aufrechterhaltung der hydrophilen Oberflächenschicht in einem benetzten Zustand während der Aufnahme im rohrförmigen Element (3) und Bereitstellung einer einsatzbereiten Katheteranordnung (1).

12. Katheteranordnung (1) nach Anspruch 10, wobei das Benetzungsfluid in einem Benetzungsfluid-Fach, das das Benetzungsfluid getrennt von der hydrophilen Oberflächenschicht des Katheters (130) während der Aufnahme im rohrförmigen Element (3) hält, und das Benetzungsfluid kann in einen Aktivierungszustand gebracht werden, um in Kontakt mit der hydrophilen Oberflächenschicht vor der beabsichtigten Verwendung gebracht zu werden.

13. Katheteranordnung (1) nach einem der vorherigen Ansprüche, wobei zumindest ein Teil der Aufnahme (2) eine Tasche (7) bildet, die das Auslassende (131) des Katheters (130) aufnimmt, wobei die Tasche (7) in Fluidkommunikation mit dem rohrförmigen Element (3) ist.

14. Katheteranordnung (1) nach Anspruch 13 in Kombination mit Anspruch 13, die ferner eine Kammer (6) umfasst, wie zum Beispiel ein Säckchen, die das Benetzungsfluid enthält, wobei die Kammer (6) in der Tasche (7) aufgenommen wird.

15. Katheteranordnung (1) nach Anspruch 14, wobei die Kammer (6) im Aktivierungszustand dafür ausgelegt ist, das Auslassende (131) des Katheters (130) zu überlappen und in dem rohrförmigen Element (3) geöffnet zu werden, das einem Ende gegenüberliegt.

16. Katheteranordnung (1) nach Anspruch 14 oder 15, wobei die Tasche (7) im Aktivierungszustand dafür ausgelegt ist, das Auslassende (131) des Katheters (130) vom Benetzungsfluid mittels einer Trennwand getrennt zu halten.

17. Katheteranordnung (1) nach einem der Ansprüche 13 bis 16, wobei die Tasche (7) im Inneren zur Verbindung des rohrförmigen Elementes (3) hin verjüngt ist, wodurch es dem Benetzungsfluid erleichtert wird, von der Tasche (7) zum rohrförmigen Element (3) übertragen zu werden.

18. Katheteranordnung (1) nach einem der Ansprüche 13 bis 17, wobei die Tasche (7) mindestens eine Öffnung (8) zum Zurückziehen des Katheters (130) vor der beabsichtigten Verwendung umfasst.

19. Katheteranordnung (1) nach Anspruch 18, wobei die Öffnung (8) eine wiederverschließbare Öffnung ist und vorzugsweise eine abziehbare Öffnung ist.

20. Katheteranordnung (1) nach Anspruch 18 oder 19, wobei die Öffnung (8) eine Aufreißöffnung ist.

21. Katheteranordnung (1) nach einem der vorherigen Ansprüche, wobei das rohrförmige Element (3) eine ablösbare Abdeckung (13) am Ende umfasst, die das Einführungsende (135) des Katheters (130) aufnimmt, wobei das restliche rohrförmige Element (14) dafür ausgelegt ist, am Auslassende (131) des Katheters (130) befestigt zu werden.

22. Katheteranordnung (1) nach einem der vorherigen Ansprüche, wobei der Katheter (130) ferner einen Verbinder (9) umfasst, wobei ein Ende des Verbinders (9) das Auslassende (131) des Katheters (130) bildet.

23. Katheteranordnung (1) nach Anspruch 22 in Kombination mit Anspruch 21, wobei das restliche rohrförmige Element (14) dafür ausgelegt ist, am Verbinder (9) befestigt zu werden.

24. Katheteranordnung (1) nach einem der vorherigen Ansprüche, die ferner eine rohrförmige Einführungshilfe (10) umfasst, die zumindest teilweise rund um mindestens einen Teil des Auslassendes (131) des Katheters (130) angeordnet ist, wobei die rohrförmige Einführungshilfe (10) dafür ausgelegt ist, nach dem Zurückziehen des Katheters (130) aus der Aufnahme (2), entlang des Katheters (130) beweglich zu sein, um dadurch als eine Einführungshilfe zum Manövrieren des benetzten Katheters (130) während der Einführung in den menschlichen Hohlraum zu fungieren.

25. Katheteranordnung (1) nach Anspruch 24, wobei die rohrförmige Einführungshilfe (10) einen Schlitz (11) entlang einer Längsrichtung hat, wobei die rohrförmige Einführungshilfe (10) dadurch leicht vom Katheter (130) gelöst werden kann, da der Schlitz (11) zum Entfernen der rohrförmigen Einführungshilfe (10) aus dem Katheter (130) genutzt werden kann.

26. Katheteranordnung (1) nach einem der vorherigen Ansprüche, wobei die Aufnahme (2) einen Urinsammelbeutel bildet.

27. Verfahren zur Aktivierung der Katheteranordnung nach einem der vorherigen Ansprüche, umfassend:
Strecken (S702) der Katheteranordnung (1) in eine ungekrümmte Anordnung,
Benetzen (S703) der hydrophilen Oberflächenschicht, und
Zurückziehen (S705) des Katheters (130) aus der Aufnahme (2).

28. Verfahren zum Herstellen einer Katheteranordnung (1), wobei die Anordnung (1) einen Blasenkatheter (130) umfasst, der ein Einführungsende (135) und ein Auslassende (131) hat, und eine Aufnahme (2), die ein rohrförmiges Element (3) umfasst, wobei das Verfahren umfasst:
Anordnen (S801) einer Vielzahl von Falten (5) in mindestens einer gefalteten Region (4) entlang des rohrförmigen Elementes (3), und
Aufnehmen (S802) eines Katheterabschnitts einschließlich des Einführungsendes (135) des Katheters (130) im rohrförmigen Element (3),
**dadurch gekennzeichnet, dass** die Falten (5) in einem Lagerzustand eine Krümmung bilden und aufrecht erhalten, wodurch sie eine gekrümmte Anordnung eines Katheterabschnitts ermöglichen, der sich darin befindet; und durch den weiteren Schritt das Umschließen der Aufnahme (2) in einem Außengehäuse (12) ermöglichen, wenn die Aufnahme (2) in der gekrümmten Anordnung des Lagerungszustandes angeordnet ist.

## Revendications

1. Ensemble de cathéter (1) comprenant un cathéter urinaire (130) doté d'une extrémité d'insertion (135) et d'une extrémité de décharge (131), ledit cathéter (130), au moins pour ladite extrémité insérable, comportant sur au moins une partie de sa surface une couche superficielle hydrophile procurant un caractère de surface à faible friction du cathéter (130) par traitement avec un fluide de mouillage, et un réceptacle (2), ledit réceptacle (2) comprenant un élément tubulaire (3) qui, en état de stockage, abrite une section de cathéter incluant l'extrémité d'insertion (135) dudit cathéter (130), ledit élément tubulaire (3) comportant au moins une zone plissée (4) située le long dudit élément tubulaire (3) et conçue avec une pluralité de plis (5), **caractérisé en ce que** lesdits plis (5), dans ledit état de stockage, forment et maintiennent une courbure, en permettant ainsi une disposition courbée d'une section de cathéter située dedans, et **en ce qu'**il comprend en outre un boîtier extérieur (12) renfermant ledit réceptacle (2) lorsque le réceptacle (2) est disposé dans ladite disposition courbée de l'état de stockage.

2. Ensemble de cathéter (1) selon la revendication 1, dans lequel ladite zone plissée (4) forme une section similaire à un soufflet dudit élément tubulaire.

3. Ensemble de cathéter (1) selon la revendication 1 ou 2, dans lequel ladite zone plissée (4) est apte à maintenir une courbe de diamètre minimal pour le cathéter (130) dans ledit état de stockage.

4. Ensemble de cathéter (1) selon l'une quelconque des revendications précédentes, dans lequel ledit ensemble de cathéter (1) comprend au moins deux et de préférence trois zones plissées séparées (4).

5. Ensemble de cathéter (1) selon l'une quelconque des revendications précédentes, dans lequel ledit élément tubulaire (3), avant son utilisation prévue, est apte à être redressé dans une disposition non courbée.

6. Ensemble de cathéter (1) selon l'une quelconque des revendications précédentes, dans lequel, dans ledit état de stockage, une longueur maximale dudit ensemble de cathéter (1) est inférieure à la moitié d'une longueur dudit cathéter (130), et de préférence à environ un tiers de la longueur dudit cathéter.

7. Ensemble de cathéter (1) selon l'une quelconque des revendications précédentes, dans lequel la longueur dudit cathéter (130) excède 200 mm, et excède de préférence 300 mm.

8. Ensemble de cathéter (1) selon l'une quelconque des revendications précédentes, dans lequel ledit élément tubulaire (3) a un diamètre intérieur seulement légèrement supérieur à un diamètre extérieur dudit cathéter (130), ledit élément tubulaire (3) créant ainsi un environnement étroit pour ledit cathéter (130).

9. Ensemble de cathéter (1) selon l'une quelconque des revendications précédentes, dans lequel ledit cathéter (130) est un cathéter urinaire destiné à une utilisation intermittente.

10. Ensemble de cathéter (1) selon l'une quelconque des revendications précédentes, comprenant en outre un fluide de mouillage.

11. Ensemble de cathéter (1) selon la revendication 10, dans lequel ledit fluide de mouillage est disposé en contact de mouillage avec ladite couche superficielle hydrophile afin de garder ladite couche superficielle hydrophile en état mouillé pendant qu'elle se trouve dans ledit élément tubulaire (3) et de créer un ensemble de cathéter prêt à utiliser (1).

12. Ensemble de cathéter (1) selon la revendication 10, dans lequel ledit fluide de mouillage est disposé dans un compartiment à fluide de mouillage gardant le fluide de mouillage séparé de ladite couche superficielle hydrophile dudit cathéter (130) pendant qu'il se trouve dans ledit élément tubulaire (3), et ledit fluide de mouillage peut être libéré dans un état d'activation pour être amené en contact avec ladite couche superficielle hydrophile avant une utilisation prévue.

13. Ensemble de cathéter (1) selon l'une quelconque des revendications précédentes, dans lequel au moins une partie dudit réceptacle (2) forme une poche (7) abritant ladite extrémité de décharge (131) dudit cathéter (130), ladite poche (7) étant en communication fluidique avec ledit élément tubulaire (3).

14. Ensemble de cathéter (1) selon la revendication 13 en combinaison avec la revendication 13, comprenant en outre un compartiment (6), tel qu'un sachet, contenant ledit fluide de mouillage, ledit compartiment (6) étant logé dans ladite poche (7).

15. Ensemble de cathéter (1) selon la revendication 14, dans lequel ledit compartiment (6) est disposé, dans ledit état d'activation, de manière à recouvrir l'extrémité de décharge (131) dudit cathéter (130) et à être ouvert dans une extrémité faisant face audit élément tubulaire (3).

16. Ensemble de cathéter (1) selon la revendication 14 ou 15, dans lequel ladite poche (7) est conçue pour, dans ledit état d'activation, garder l'extrémité de décharge (131) dudit cathéter (130) séparée dudit fluide de mouillage au moyen d'une cloison de séparation.

17. Ensemble de cathéter (1) selon l'une quelconque des revendications 13 à 16, dans lequel ladite poche (7) se rétrécit vers l'intérieur vers le raccord dudit élément tubulaire (3), en facilitant ainsi le transfert dudit fluide de mouillage de ladite poche (7) vers ledit élément tubulaire (3).

18. Ensemble de cathéter (1) selon l'une quelconque des revendications 13 à 17, dans lequel ladite poche (7) comprend au moins une ouverture (8) pour le retrait dudit cathéter (130) avant l'utilisation prévue.

19. Ensemble de cathéter (1) selon la revendication 18, dans lequel ladite ouverture (8) est une ouverture rescellable et de préférence une ouverture pelable.

20. Ensemble de cathéter (1) selon la revendication 18 ou 19, dans lequel ladite ouverture (8) et une ouverture déchirable.

21. Ensemble de cathéter (1) selon l'une quelconque des revendications précédentes, dans lequel ledit élément tubulaire (3) comprend un recouvrement détachable (13) à son extrémité abritant ladite extrémité d'insertion (135) du cathéter (130), l'élément tubulaire résiduel (14) étant apte à être rattaché à ladite extrémité de décharge (131) du cathéter (130).

22. Ensemble de cathéter (1) selon l'une quelconque des revendications précédentes, dans lequel le cathéter (130) comprend en outre un connecteur (9), une extrémité dudit connecteur (9) formant ladite extrémité de décharge (131) du cathéter (130).

23. Ensemble de cathéter (1) selon la revendication 22 en combinaison avec la revendication 21, dans lequel ledit élément tubulaire résiduel (14) est conçu pour être rattaché audit connecteur (9).

24. Ensemble de cathéter (1) selon l'une quelconque des revendications précédentes, comprenant en outre une aide à l'insertion tubulaire (10) disposée au moins partiellement autour d'au moins une partie de ladite extrémité de décharge (131) du cathéter (130), ladite aide à l'insertion tubulaire (10) étant disposée de manière à, après le retrait dudit cathéter (130) dudit réceptacle (2), être mobile le long dudit cathéter (130) pour faire ainsi office d'aide à l'insertion pour manoeuvrer le cathéter mouillé (130) pendant son insertion dans la cavité humaine.

25. Ensemble de cathéter (1) selon la revendication 24, dans lequel ladite aide à l'insertion tubulaire (10) comporte une fente (11) dans un sens longitudinal, ladite aide à l'insertion tubulaire (10) étant ainsi facilement détachable dudit cathéter (130) du fait que ladite fente (11) peut être utilisée pour retirer ladite aide à l'insertion tubulaire (10) dudit cathéter (130).

26. Ensemble de cathéter (1) selon l'une quelconque des revendications précédentes, dans lequel ledit réceptacle (2) forme un sac de collecte d'urine.

27. Procédé d'activation de l'ensemble de cathéter selon l'une quelconque des revendications précédentes, comprenant :
le redressement (S702) dudit ensemble de cathéter (1) en une disposition non courbée,
le mouillage (S703) de ladite couche superficielle hydrophile, et
le retrait (S705) dudit cathéter (130) dudit réceptacle (2).

28. Procédé de fabrication d'un ensemble de cathéter (1), lequel ensemble (1) comprend un cathéter urinaire (130) doté d'une extrémité d'insertion (135) et d'une extrémité de décharge (131), et un réceptacle (2) comprenant un élément tubulaire (3), ledit procédé comprenant :
la disposition (S801) d'une pluralité de plis (5) dans au moins une zone plissée (4) le long dudit élément tubulaire (3), et
le logement (S802) d'une section de cathéter incluant l'extrémité d'insertion (135) dudit cathéter (130) dans ledit élément tubulaire (3),
**caractérisé en ce que** lesdits plis (5), en état de stockage, forment et maintiennent une courbure en permettant ainsi une disposition courbée d'une section de cathéter se trouvant dedans ; et par l'autre étape consistant à renfermer le réceptacle (2) dans un boîtier extérieur (12) lorsque le réceptacle (2) est disposé dans ladite disposition courbée de l'état de stockage.
